# FASCICULE DE BREVET EUROPEEN

(11) **EP 1 176 928 B1**
(45) Date de publication et mention de la délivrance du brevet: **20.07.2005**
(21) Numéro de dépôt: 00925369.1
(22) Date de dépôt: 02.05.2000
(51) Int. Cl.: A61F 2/16

(54) **IMPLANT DE CHAMBRE POSTERIEURE APHAKE CHEZ LE MYOPE FORT**
APHAKES HINTERKAMMERIMPLANTAT FÜR STARK KURZSICHTIGE
APHAKIC POSTERIOR CHAMBER IMPLANT FOR HIGH MYOPIA

(30) Priorité: 30.04.1999 FR 9905547
(43) Date de publication de la demande: 06.02.2002
(73) Titulaire: Ioltechnologie-Production, 17000 La Rochelle (FR)
(72) Inventeur: CHASSAIN, Christophe, F-34980 Saint Clément de Rivière (FR)
(74) Mandataire: Santarelli
(86) Numéro de dépôt international: PCT/FR2000/001177
(87) Numéro de publication internationale: WO 2001/006955

(56) Documents cités:
- EP-A- 0 064 770
- EP-A- 0 579 528
- EP-A- 0 698 381
- WO-A-97/43984
- FR-A- 2 700 466
- FR-A- 2 745 711
- SU-A- 1 553 109
- US-A- 4 738 680

## Description

L'invention concerne un implant intraoculaire de chambre postérieure de l'oeil pour aphake, notamment chez le myope fort.

La myopie forte correspond à une longueur axiale optique supérieure à 26 mm. La cataracte chez ces sujets est plus précoce, vers 55 ans, que pour la population générale et pose des problèmes particuliers qui n'ont pas encore été résolus.

L'implant intraoculaire, également appelé lentille intraoculaire, comporte une partie optique centrale et une partie haptique périphérique et est destiné à être implanté dans l'oeil aphake, c'est-à-dire après extraction du cristallin atteint d'une cataracte.

Classiquement, les implants ou lentilles intraoculaires destinés aux myopes forts étaient réalisés en matériau rigide, tel que le polymethacrylate de méthyle (PMMA) et la partie optique biconvexe de grand diamètre, de 6,5 mm à 7 mm, le rayon de courbure de la face postérieure convexe étant compris entre 20 et 30 mm. Après ablation du cristallin, le corps vitré n'était plus maintenu et était particulièrement instable chez le myope fort. Une grande optique en matériau rigide est censée assurer une bonne stabilité du corps vitré afin d'éviter les risques de lésion rétinienne. Les éléments haptiques destinés au support de l'implant dans le sac capsulaire ou sulcus ciliaire sont constitués d'anses en forme de C. Plus récemment, on a conçu de tels implants intraoculaires à optique biconvexes, destinés aux myopes forts, en matériau souple, notamment hydrophobe, également avec des anses en forme de C. On remarque que des anses en forme de C sont préférées aux haptiques dites plates, puisque mieux à même à s'adapter à des grandes variations supposées de diamètre du sac capsulaire chez le myope fort.

Le brevet FR-A-2.700.466 décrit un implant intraoculaire destiné au myope fort, réalisé en matériau rigide qui a une partie optique concave-convexe, également de grand diamètre, supérieur à 7 mm, avec un rayon de courbure de la face postérieure convexe supérieure à 12 mm. Les éléments optiques sont constitués par les anses en forme de C.

Tous ces implants présentent des inconvénients sérieux. D'abord, en ce qui concerne les implants en matériau rigide avec une optique de grand diamètre, l'incision doit être au moins aussi longue que le diamètre de l'optique. Il y a également un risque de désinsertion zonulaire, étant donné que l'élément haptique sous forme d'anse en C est très rigide et fortement comprimé lors de l'implantation.

D'autres problèmes peuvent fréquemment survenir dans les suites opératoires d'implantation des implants en matériau rigide ayant une optique concave-convexe de grand diamètre ou en matériau souple ayant une optique biconvexe également de grand diamètre. D'abord, la fibrose capsulaire est favorisée par l'âge relativement jeune des myopes forts, dont les cellules épithéliales antérieures et équatoriales ont une activité mitotique élevée. L'adhérence des capsules antérieure et postérieure appelée "symphyse" facilitant la migration de cellules germinatoires est favorisée par des implants classiques en raison du grand rayon de courbure de la face de l'optique, et de la faible épaisseur de l'optique liée à leur faible puissance dioptrique. Etant donné l'inadéquation de taille entre le sac capsulaire et l'implant, notamment à anses en C, des plis se forment sur la partie centrale de la capsule postérieure qui constituent des réservoirs de cellules migratrices. De même, l'aphakie ou pseudo-phakie augmente le risque de décollement de la rétine de 2 % à 15 % pour des yeux dont la longueur axiale est supérieure à 26 mm dû, selon certains spécialistes, au déplacement du corps vitré vers l'avant.

Le document EP 0 698 381 décrit un implant intraoculaire réalisé en une seule pièce en PMMA, un polymère thermoplastique, silicone ou acrylique. L'optique est réalisée sous la forme d'un ménisque, c'est-à-dire concave, convexe, voire plan convexe. Mais la face concave est la face postérieure pour réduire le contact entre la capsule postérieure et elle. Le diamètre de l'optique étant approximativement égal à 5,5 mm et l'épaisseur des bras haptiques étant approximativement égale à 0,5 mm, l'encombrement axial de l'optique de la lentille est relativement important mais décalé antérieurement.

La présente invention a pour but de pallier ces inconvénients et de proposer un implant intraoculaire de chambre postérieure pour aphake, notamment chez le myope fort. A cette fin, l'implantation de la lentille intraoculaire est conçue pour assurer à la fois l'éloignement des capsules l'une de l'autre et le refoulement du corps vitré vers l'arrière.

Conformément à un aspect de l'invention, la distance axiale entre le centre de la face postérieure, convexe, de l'élément optique et la face antérieure concave ou plane à la périphérie de l'optique à la périphérie est supérieure à 0,8 mm, et plus particulièrement à 1,20 mm, et de préférence entre environ 1,20 et 1,28 mm ou entre environ 1,24 et 1,74 mm. Cette distance axiale, conforme à un aspect de l'invention, est donc très supérieure à celle des implants connus pour myopes forts de l'état de la technique qui sont, le plus souvent.

Grâce à cet encombrement axial important, l'implant assure l'éloignement des capsules antérieure et postérieure l'une de l'autre. Elles ne peuvent s'accoler qu'à la périphérie du sac, ce qui aura comme conséquence le retard de la migration cellulaire vers le centre de l'optique.

Selon une première forme de réalisation, la partie optique a également une face antérieure concave ou éventuellement plane pour le cas limite et une valeur de dioptrie entre -5 et + 15. Du fait de la concavité antérieure de la partie optique pour la quasi-totalité des implants de cette famille, on diminue le contact entre le matériau constitutif de l'implant et la capsule antérieure, par rapport à un élément optique biconvexe.

Selon une deuxième forme de réalisation, la partie optique a également une face antérieure concave et une valeur de dioptrie entre - 10 et +10.

De même, de préférence, la partie optique comporte trois anses à contour fermé, appelées également haptiques plates fenêtrées, chacune comportant deux bras s'étendant globalement radialement et une bande circonférentielle dont l'étendue angulaire est supérieure ou égale à 60° et de préférence de l'ordre de 80°.

De tels éléments haptiques élargis à leur périphérie extérieure offrent un appui supplémentaire de sécurité et diminuant le risque d'expulsion de l'implant vers l'antérieur. Cette forme des éléments haptiques amortit la contrainte exercée sur l'optique concave-convexe, réalisé en matériau souple dont le risque de déformation est très supérieur à celui de l'optique biconvexe réalisé dans le même matériau. La tension du sac est répartie d'une manière harmonieuse pour être équilibrée par les éléments haptiques qui, de préférence, sont disposés d'une manière uniforme autour de la périphérie de la partie optique. On limite ainsi le risque de formation de plis traversant la région centrale de la capsule postérieure.

En pratique, l'implant est monobloc et réalisé de préférence en matériau acrylique hydrophile ou d'autres matériaux dits souples, tels que hydrogel, polyHEMA, silicone ou acrylique hydrophobe, ce qui permet à l'implant d'être plié ou roulé et introduit dans l'oeil par une incision de 5 mm, voire 3,5 mm, avec un injecteur. Ceci permet l'introduction par la voie cornéenne et une fermeture rapide sans désinsertion conjonctivale.

Enfin, grâce à la forme des éléments haptiques, le passage de l'implant dans le rhexis se fait sans tension zonulaire excessive, d'une part et élimine toute possibilité de capture de l'optique par la capsule antérieure même avec un rhexis de même diamètre que celui de l'élément optique.

L'invention sera mieux comprise et d'autres avantages de celle-ci apparaîtront à la lumière de la description qui va suivre, donnée à titre d'exemple et faite en référence aux dessins annexés sur lesquels :
- la figure 1 est une vue de face d'un implant ou lentille intraoculaire de chambre postérieure conforme à un mode de réalisation de l'invention ;
- la figure 2 est une vue en coupe de l'implant ou lentille de la figure 1 dont l'élément optique est de forme concave-convexe ;
- la figure 3 est une vue en coupe d'un oeil illustrant l'implantation de l'implant des figures 1 et 2 ;
- la figure 4 est une vue en coupe de l'implant de la figure 1 dont l'élément optique est plan-convexe ;
- la figure 5 est une vue analogue à celle de la figure 1 pour un second mode de réalisation;
- la figure 6 est une vue en coupe de l'implant de la figure 5 dont la valeur de dioptrie de l'optique est nettement négative;
- la figure 7 est une vue en coupe de l'impolant de la figure 5 dont la valeur de dioptrie de l'optique est positive; et
- la figure 8 est une vue correspondant à celle de la figure 6 pour illustrer l'implantation de la lentille dans le sac capsulaire.

L'implant comporte une partie optique et une partie haptique. Selon le premier mode de réalisation, la partie optique est constituée d'un élément optique central 10 ayant une face antérieure concave et une face postérieure convexe 12, et la partie haptique d'une pluralité d'éléments haptiques périphériques 20 qui s'étendent radialement vers l'extérieur à partir de la périphérie de l'élément optique 10.

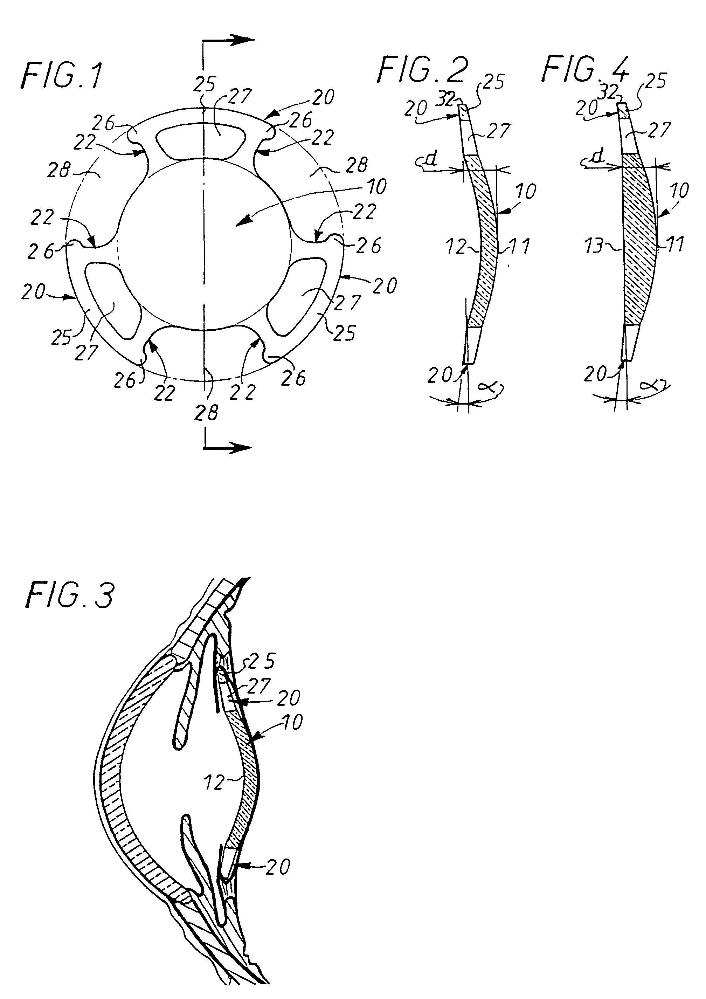

S'agissant d'un implant destiné à un sujet ayant une forte myopie, la forme de l'élément optique est concave-convexe tel qu'illustré sur la figure 2, ou éventuellement plan-convexe pour le cas limite (dioptrie + 15), tel qu'illustré à la figure 3. Dans tous les cas, la face postérieure 1 2 est convexe, la face antérieure 11 étant concave (figure 2), ou plane 13 (figure 3), selon le cas.

De préférence, le rayon de courbure de la face postérieure convexe pour myope fort de l'état de la technique est relativement faible par rapport au rayon de courbure des implants mais supérieur au rayon du cristallin et par exemple de l'ordre de 8,5 mm. Le rayon de courbure est constant pour toute la gamme des implants destinés au myope fort pour des puissances de -5 à + 15 dioptries selon ce premier mode de réalisation. Le diamètre de l'élément optique 10 est supérieur à 6 mm et de préférence à 6,5 mm. Avec le même rayon de courbure de la face postérieure d'environ 8,5 mm, on obtient la correction réfractive pour les dioptries indiquées, tout en gardant une face antérieure de l'optique concave ou éventuellement plane pour le cas limite.

L'épaisseur de l'élément optique 10 au centre optique est en pratique inférieure à 0,6 mm et de préférence 0,6 mm pour les dioptries négatives, et jusqu'à 1,25 mm pour la dioptrie positive la plus importante, à savoir + 15.

L'épaisseur axiale de l'élément optique 10 à sa périphérie est en pratique également supérieure à 0,5 mm et de préférence entre environ 0,6 mm pour des dioptries positives, et jusqu'à 0,75 mm pour la dioptrie négative la plus importante, à savoir -5.

Selon l'invention, la distance axiale d définie entre le centre de la face postérieure et la face antérieure à la périphérie de l'optique est supérieure à 0,8 mm et plus particulièrement supérieure à environ 1,20 mm et de préférence entre 1,20 mm et 1,28 mm pour cette forme de réalisation. On assure ainsi un bon écartement de la partie restante, appelée rhexis, de la capsule antérieure, de la capsule postérieure intacte.

La partie haptique comporte une pluralité d'éléments haptiques 20 s'étendent vers l'extérieur à partir de la périphérie de l'élément optique 10 Ces éléments haptiques 20 sont du type à anse fermée également appelée haptique plate fenêtrée.

Tel qu'illustré, la partie haptique comporte trois éléments haptiques 20 de même forme et dimensions 20 régulièrement espacés autour de la périphérie de l'élément optique, à 120° les uns des autres. Bien que cette disposition assure la meilleure stabilité, on pourra adopter d'autres dispositions, notamment une symétrie diamétrale avec deux des éléments haptiques plus rapprochés, ou quatre éléments haptiques, ou même deux éléments haptiques disposés à 180° l'un de l'autre, chacun avec une étendue circonférentielle de 90°.

Chacun des éléments haptiques 20 comporte deux bras 22 s'étendant globalement radialement, et une bande circonférentielle 25 coaxiale avec l'élément optique et joignant les bras radiaux de l'élément haptique. L'étendue circonférentielle de chaque bande circonférentielle 25 est de préférence au moins de 60° de sorte que l'étendue de l'appui des éléments haptiques est au total de 180° à l'équateur du sac capsulaire.

Le bord périphérique 32 de la bande circonférentielle 25 est délimité par des arêtes, ce qui assure la meilleure barrière contre la migration des cellules germinatoires.

Avec cette disposition des haptiques, toute ligne diamétrale de la lentille passe par une des haptiques.

Selon une variante non représentée, les bandes circonférentielles constituent des segments d'un anneau périphérique continu, d'autres segments joignant les bandes à leurs extrémités 26.

Tel qu'illustré, les extrémités 26 de la bande circonférentielle s'étendent au-delà des bras radiaux 22 dans le sens circonférentiel par rapport à l'extrémité distale des bras à la jonction avec le bord périphérique de l'élément optique.

A leur extrémité proximale, chaque bras radial 22 est très évasé et à leur extrémité distale au niveau de la jonction avec la bande, la largeur circonférentielle est légèrement réduite. La largeur moyenne des bras radiaux, en pratique environ 0,6 mm, est sensiblement la même que l'épaisseur axiale des bras à l'extrémité proximale 24.

La fenêtre 27 à l'intérieur de chaque élément haptique plat a un contour trapézoïdal, les côtés intérieurs et extérieurs concentriques étant respectivement confondus avec le bord périphérique de l'élément optique 10 et le bord intérieur de la bande circonférentielle 25, et les côtés latéraux avec le bord intérieur des bras radiaux 22 de l'élément haptique.

L'épaisseur axiale des éléments haptiques 20 diminue progressivement de l'extrémité proximale des bras radiaux jusqu'au bord périphérique extérieur de la bande circonférentielle 25. De préférence, l'épaisseur axiale au bord périphérique extérieur de la bande circonférentielle est supérieure à 30 mm, et 0,35 mm dans la forme de réalisation préférée pour des dioptries positives et entre 0,35 mm et 0,5 mm pour des dioptries négatives. De même, une épaisseur d'environ 0,6 mm est préférée au niveau de la jonction optique pour des dioptries positives et entre 0,6 mm et 0,75 mm pour des dioptries négatives.

Les éléments haptiques ont de préférence une faible angulation α vers l'antérieure inférieure à 10°, et plus particulièrement de l'ordre de 5 °, par rapport à la perpendiculaire de l'axe optique.

D'autres configurations sont également envisageables et notamment une pente plus raide à partir de l'extrémité proximale des bras radiaux suivis d'une zone d'épaisseur sensiblement constante.

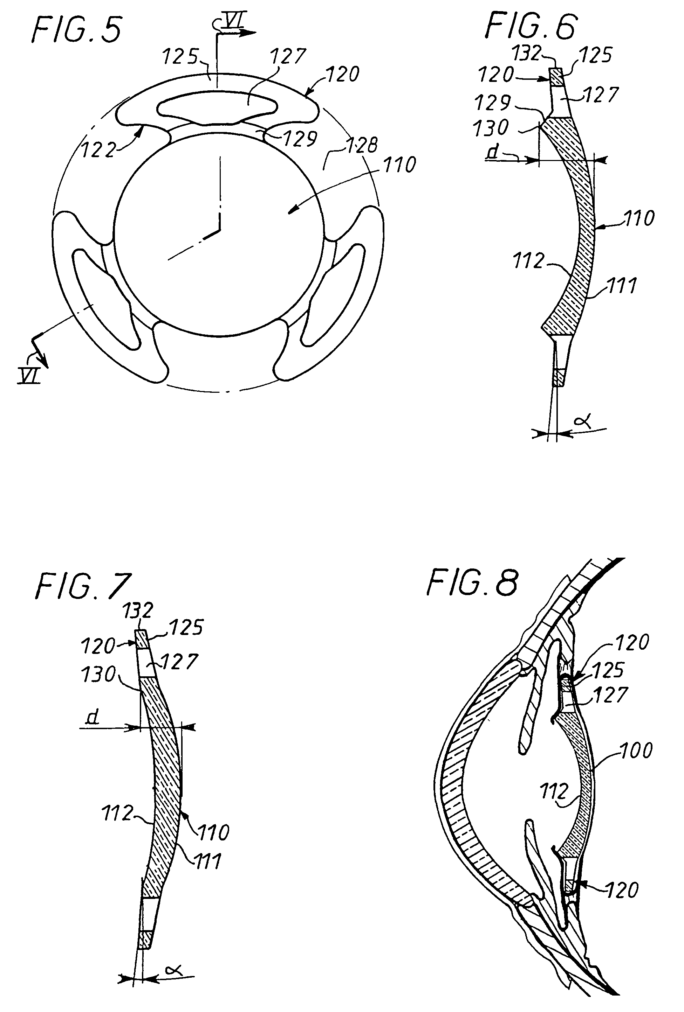

L'implant selon le second mode de réalisation illustré aux figures 5 à 8 a la même architecture générale que celui du premier mode de réalisation. Les parties correspondantes sont désignées par des références augmentées d'une centaine. L'élément optique central 110 a une face postérieure convexe 112, une face antérieure concave 111, et une partie haptique comprenant une pluralité d'éléments haptiques périphériques 120.

De préférence, le rayon de courbure de la face postérieure convexe 112 est de l'ordre de 8,5 mm. Le rayon de courbure est constant pour toute la gamme des implants pour des puissances de -10 à + 10 dioptries selon cette seconde forme de réalisation. Le diamètre de l'élément optique 110 est, comme pour la première forme de réalisation, supérieur à 6 mm et de préférence à 6,5 mm.

L'épaisseur de l'élément optique 110 au centre de l'optique est d'au moins 0,85 mm pour les dioptries les plus négatives et de 0,50 mm pour une valeur de dioptrie de -10. L'épaisseur axiale de l'élément optique 110 à sa périphérie est en pratique de 0,6 mm pour des dioptries positives et jusqu'à 0,85 mm pour des dioptries modérément négatives, entre -1 et -5, et jusqu'à 1,10 mm pour les dioptries les plus négatives de la gamme.

La distance axiale d définie entre le centre de la face postérieure et la face antérieure à la périphérie de l'optique, pour ce second mode de réalisation, est supérieur à 1,20 mm et de préférence égale ou supérieure à 1,24 mm, et plus particulièrement 1,24 mm pour toutes les puissances positives et entre 1,24 mm et 1,74 mm pour les puissances négatives.

Tel qu'illustré, la partie haptique comporte trois éléments haptiques 120 du même type, c'est-à-dire à anses fermées, appelés également haptiques plates fenêtrées, ces éléments étant régulièrement espacés à 120° les uns des autres. Bien que cette disposition assure la meilleure stabilité, on pourra adopter d'autres dispositions, notamment une symétrie diamétrale avec deux des éléments haptiques plus rapprochés, ou quatre éléments haptiques.

Chacun des éléments haptiques 120 comporte deux bras 122 s'étendant globalement radialement, et une bande circonférentielle 125 coaxiale avec l'élément optique et joignant les bras radiaux de l'élément haptique. L'étendue circonférentielle de chaque bande circonférentielle 1 25 est de préférence au moins de 80° de sorte que l'étendue de l'appui des éléments haptiques est au total de 240° à l'équateur du sac capsulaire. Le bord périphérique 132 de la bande circonférentielle 125 est délimité par des arêtes.

Avec cette disposition des haptiques, toute ligne diamétrale de la lentille passe au moins par une des haptiques, selon une variante non illustrée. Les bandes circonférentielles peuvent également constituer des segments d'un anneau périphérique continu.

A leur extrémité proximale, les bras radiaux des éléments haptiques 122 se joignent et forment un segment annulaire commun 129. De préférence, l'étendue angulaire de ce segment annulaire est d'environ 60°. Sur la face antérieure de l'implant, ce segment annulaire forme une surface tronconique à partir de l'arête 130 à la périphérie de l'élément optique 110. L'inclinaison de cette surface tronconique est beaucoup plus forte que l'angle α d'angulation des éléments haptiques dont la valeur sera la même que dans la première forme de réalisation. La distance radiale de ce segment annulaire 129 varie en fonction de la dioptrie de la partie optique concave-convexe. Pour les dioptries négatives, elle est de l'ordre de 0,5 mm et de 0,25 mm pour les dioptries négatives plus faibles. Pour les dioptries positives, tel qu'illustré à la figure 7, la pente du segment annulaire est la même que l'angulation des haptiques.

La fenêtre 127 à l'intérieur de chaque élément haptique plat 120 a également un contour général trapézoidal mais plus aplati et plus large que celui du premier mode de réalisation avec une étendue angulaire de 60° environ et une largeur radiale au centre de l'ordre de 2 mm. Dans ce mode de réalisation, le côté intérieur concentrique de la fenêtre se confond non pas avec le bord périphérique de l'élément optique 110 mais avec le bord périphérique du segment annulaire, et les côtés latéraux de la fenêtre ont une étendue circonférentielle beaucoup plus marquée que dans le premier mode de réalisation.

Comme dans le premier mode de réalisation, l'épaisseur axiale des éléments haptiques 120 diminue progressivement vers la périphérie extérieure de la bande circonférentielle 125. De même, l'épaisseur axiale au bord périphérique extérieur de la bande circonférentielle est supérieure à 0,30 mm, et 0,35 mm dans la forme de réalisation préférée tant pour des dioptries positives que pour des dioptries négatives.

Quel que soit le mode de réalisation, l'implant est de préférence monobloc et entièrement réalisé en un matériau dit souple qui permet à l'implant d'être plié ou roulé et ainsi permettre de l'introduire à travers une incision cornéenne de 5 mm, voire 3,5 mm si l'introduction est effectuée avec un injecteur, et en tout cas bien inférieure au diamètre de l'élément optique qui sera supérieur à 6 mm, qui serait celui nécessaire pour l'implantation d'un implant concave-convexe de l'état de la technique où l'implant est réalisé en un matériau rigide, tel que le PMMA.

L'élément optique d'un diamètre supérieur à 6 mm et de préférence de l'ordre de 6,5 mm, une éventuelle intervention ultérieure du rétinologue, hélas fréquente pour le myope fort, est rendue plus aisée.

D'une manière avantageuse, avec un tel implant le rhexis peut avoir un diamètre sensiblement égal au diamètre du l'élément optique sans augmenter le risque d'une capture de l'élément optique par le bord libre du rhexis et l'expulsion dans la chambre. Plusieurs caractéristiques concourent à cet avantage. D'abord le positionnement et le nombre (6, selon la forme de réalisation préférée) des bras radiaux des éléments haptiques autour de la périphérie de l'optique, et surtout leur étendue angulaire à leur extrémité proximale est de l'ordre de 60°, voire davantage pour le premier mode de réalisation. La forme des éléments haptiques avec la grande étendue circonférentielle totale (environ 180° dans le premier mode de réalisation et 240° dans le second mode de réalisation) assure une assise stable et sûre de l'implant dans le sac capsulaire. Enfin, la forme des éléments haptiques et le diamètre de l'implant pris au niveau du bord extérieur des éléments haptiques de 10,5 mm, voire légèrement inférieur, réduit la contrainte élastique exercée sur l'optique après rétraction du sac capsulaire. De ce fait, les haptiques sont peu déformées par rapport à leur configuration au repos et la contrainte élastique peu élevée, juste assez pour assurer une position stable dans le sac capsulaire.

La configuration de l'implant selon l'invention s'oppose à la formation de plis dans la partie centrale de la capsule postérieure, source de propagation de fibrose sur toute l'étendue des plis.

Grâce à l'architecture de l'implant selon l'invention, les capsules postérieures restent écartées au niveau de la périphérie de l'élément optique. La zone de contact entre les capsules est en pratique limitée à la partie périphérique de l'échancrure 28, 128 entre des éléments haptiques adjacents 20, 120 et s'étendant globalement tangentiellement à partir des extrémités des bandes circonférentielles des deux éléments haptiques adjacents, et donc relativement éloignée du bord de l'élément optique.

Le rayon de courbure de la face postérieure convexe de l'élément optique est de préférence relativement petit, de l'ordre de 8,5 mm, mais plus grand que le rayon de courbure de la face postérieure du cristallin naturel (environ 6 mm), qui présente à la fois une bonne résistance à la pression exercée par le corps vitré antérieurement et surtout une courbure permettant d'adopter des éléments optiques concaves-convexes, ou plans-convexes pour le cas limite, pour les dioptries entre -5 et + 15 du premier mode de réalisation, et -10 et + 10 pour le second mode de réalisation.

Enfin, l'architecture de l'implant permet d'utiliser un matériau souple et de préférence un acrylique hydrophile pour un grand diamètre de l'élément supérieur à 6 mm et de préférence de l'ordre de 6,5 mm et une configuration concave-convexe ou plan-convexe, tout deux très avantageux pour des implants destinés aux myopes forts.

On comprendra que l'architecture générale de l'implant de l'invention pourra convenir pour des implants qui n'ont pas un élément optique concave-convexe ou plan convexe mais biconvexe (non illustrée). Une telle configuration d'implant permet une correction réfractive de + 15 à + 30 dioptries. Comme les implants destinés aux myopes forts déjà décrits et illustrés, il assure l'écartement des capsules antérieure et postérieure, pour retarder le développement de la fibrose au niveau de l'élément optique et étant donné la grande épaisseur axiale au niveau de la périphérie de l'élément optique, il réduit le risque de capture de l'élément optique et l'expulsion de l'implant vers la chambre antérieure et assure une assise stable et un bon maintien de l'implant dans le sac capsulaire.

On comprendra que la présente invention n'est pas limitée à l'ensemble des caractéristiques décrites et illustrées des formes de réalisation préférées mais au contraire s'étend à toutes les variantes et équivalents et comprises dans la portée de l'invention définies par les revendications qui suivent.

## Revendications

1. Lentille intraoculaire pour implantation dans le sac capsulaire ayant une partie optique centrale (10, 110) à face postérieure convexe (11,111) et à face antérieure concave ou plan et une partie haptique périphérique (20), **caractérisée en ce que** la partie optique est réalisée en matériau souple et la distance axiale d entre le centre de la face postérieure de la partie optique et la surface antérieure à la périphérie de la partie optique est supérieure à 0,8 mm.

2. Lentille intraoculaire selon la revendication 1, **caractérisée en ce** la valeur de dioptrie de la partie optique est entre -5 et + 15.

3. Lentille intraoculaire selon la revendication 1, **caractérisée en ce que** la valeur de dioptrie de la partie optique est entre -10 et + 10.

4. Lentille intraoculaire selon l'une quelconque des revendications précédentes, **caractérisée en ce que** la distance axiale est supérieure à environ 1,20.

5. Lentille intraoculaire selon l'une quelconque des revendications 1 à 4, **caractérisée en ce que** la distance axiale est entre environ 1,20 et 1,28 mm.

6. Lentille intraoculaire selon l'une quelconque des revendications 1 à 4, **caractérisée en ce que** la distance axiale est égale ou supérieure à 1,24 mm.

7. Lentille intraoculaire selon l'une quelconque des revendications 1 à 4, **caractérisée en ce que** la distance axiale est entre environ 1,24 mm et 1,74 mm.

8. Lentille intraoculaire selon l'une quelconque des revendications précédentes, **caractérisée en ce que** la partie optique est réalisée entièrement en matériau souple.

9. Lentille intraoculaire selon l'une quelconque des revendications précédentes, **caractérisée en ce que** le diamètre de la partie optique est entre 6 et 7 mm environ et le diamètre défini par la périphérie de la partie haptique est de 10,5 ± 0,5 mm.

10. Lentille intraoculaire selon l'une quelconque des revendications précédentes, **caractérisée en ce que** la partie haptique (20, 120) comporte trois anses à contour fermé, chacune comportant deux bras (22,122) s'étendant globalement radialement et une bande circonférentielle (25, 125) dont l'étendue angulaire est égale ou supérieure à 60°.

11. Lentille intraoculaire selon l'une quelconque des revendications 1 à 9, **caractérisée en ce que** la partie haptique (120) comporte trois anses à contour fermé, chacune comportant deux bras (122) s'étendant globalement radialement et une bande circonférentielle (125) dont l'étendue angulaire est de l'ordre de 80°.

12. Lentille intraoculaire selon la revendication 10 ou 11, **caractérisée en ce que** les bandes circonférentielles des anses constituent des segments (129) d'un anneau périphérique d'autres segments liant les trois bandes circonférentielles entre elles.

13. Lentille intraoculaire selon la revendication 10, 11 ou 12, **caractérisée en ce que** l'étendue angulaire de la partie haptique (20, 120) à la périphérie de la partie optique est égale, voire supérieure à 60°

14. Lentille intraoculaire selon l'une quelconque des revendications 10 à 13, **caractérisée en ce que** les trois anses sont identiques et présentent un angle de 120° les unes par rapport aux autres.

15. Lentille intraoculaire selon l'une quelconque des revendications précédentes, **caractérisée en ce que** le rayon de courbure de la face postérieure est supérieur à environ 6 mm.

16. Lentille intraoculaire selon la revendication 15, **caractérisée en ce que** le rayon de courbure de la face postérieure est de l'ordre de 8,5 mm.

17. Lentille intraoculaire selon l'une quelconque des revendications précédentes, **caractérisée en ce que** le diamètre de la partie optique est de l'ordre de 6,5 mm.

18. Une famille de lentilles intraoculaires selon l'une quelconque des revendications 1 à 17, **caractérisée en ce que** chaque lentille de la famille a sensiblement la même distance axiale d pour toutes les puissances entre -5 et + 15 dioptries.

19. Une famille de lentilles intraoculaires selon l'une quelconque des revendications 1 à 17, **caractérisée en ce que** chaque lentille de la famille a sensiblement la même distance axiale d pour toutes les puissances entre 0 et + 10 dioptries.

20. Une famille de lentilles intraoculaires selon la revendication 19, **caractérisée en ce que** chaque lentille de la famille a sensiblement la même distance axiale d variable pour toutes les puissances entre -10 et -1 dioptries, respectivement entre environ 1,74 mm et 1,24 mm.

## Patentansprüche

1. Intraokularlinse fur die Implantation in den Kapselsack, die einen mittigen optischen Abschnitt (10, 110) mit konvexer Ruckseite (11, 111) und konkaver oder ebener Vorderseite sowie einen haptischen Umfangsabschnitt (20) besitzt, **dadurch gekennzeichnet, dass** der optische Abschnitt aus einem nachgiebigen Werkstoff hergestellt ist und der axiale Abstand d zwischen dem Zentrum der Ruckseite des optischen Abschnitts und der vorderen Flache am Umfang des optischen Abschnitts großer als 0,8 mm ist.

2. Intraokularlinse nach Anspruch 1, **dadurch gekennzeichnet, dass** der Dioptriewert des optischen Abschnitts zwischen -5 und +15 hegt

3. Intraokularhnse nach Anspruch 1, **dadurch gekennzeichnet, dass** der Dioptriewert des optischen Abschnitts zwischen -10 und +10 liegt.

4. Intraokularlinse nach einem der vorhergehenden Anspruche, **dadurch gekennzeichnet, dass** der axiale Abstand großer als etwa 1,20 ist.

5. Intraokularlinse nach einem der Anspruche 1 bis 4, **dadurch gekennzeichnet, dass** der axiale Abstand im Bereich von etwa 1,20 bis etwa 1,28 mm liegt.

6. Intraokularhnse nach einem der Anspruche 1 bis 4, **dadurch gekennzeichnet, dass** der axiale Abstand gleich oder großer als 1,24 mm ist.

7. Intraokularlinse nach einem der Anspruche 1 bis 4, **dadurch gekennzeichnet, dass** der axiale Abstand im Bereich von etwa 1,24 mm bis etwa 1,74 mm liegt

8. Intraokularlinse nach einem der vorhergehenden Anspruche, **dadurch gekennzeichnet, dass** der optische Abschnitt vollstandig aus einem nachgiebigen Werkstoff hergestellt ist.

9. Intraokularlinse nach einem der vorhergehenden Anspruche, **dadurch gekennzeichnet, dass** der Durchmesser des optischen Abschnitts im Bereich von etwa 6 bis etwa 7 mm hegt und der durch den Umfang des haptischen Abschnitts definierte Durchmesser 10,5 ± 0,5 mm betragt.

10. Intraokularlinse nach einem der vorhergehenden Anspruche, **dadurch gekennzeichnet, dass** der haptische Abschnitt (20, 120) drei Griffe mit geschlossenem Umriss umfasst, wovon jeder zwei Arme (22, 122), die sich im Allgemeinen radial erstrecken, und ein Umfangsband (25, 125), dessen Winkelerstreckung gleich oder großer als 60° ist, aufweist

11. Intraokularlinse nach einem der Anspruche 1 bis 9, **dadurch gekennzeichnet, dass** der haptische Abschnitt (120) drei Griffe mit geschlossenem Umriss aufweist, wovon jeder zwei Arme (122), die sich im Allgemeinen radial erstrecken, und ein Umfangsband (125), dessen Winkelerstreckung in der Großenordnung von 80° liegt, aufweist.

12. Intraokularlinse nach Anspruch 10 oder 11, **dadurch gekennzeichnet, dass** die Umfangsbänder der Griffe Segmente (129) eines Umfangsrings aus anderen Segmenten, die die drei Umfangsbander untereinander verbinden, bilden.

13. Intraokularlinse nach Anspruch 10, 11 oder 12, **dadurch gekennzeichnet, dass** die Winkelerstreckung der haptische Abschnitt (20, 120) am Umfang des optischen Abschnitt gleich oder sogar großer als 60° ist

14. Intraokularlinse nach einem der Anspruche 10 bis 13, **dadurch gekennzeichnet, dass** die drei Griffe gleich sind und untereinander einen Winkel von 120° bilden

15. Intraokularlinse nach einem der vorhergehenden Anspruche, **dadurch gekennzeichnet, dass** der Krummungsradius der Ruckseite großer als etwa 6 mm ist

16. Intraokularlinse nach Anspruch 15, **dadurch gekennzeichnet, dass** der Krummungsradius der Ruckseite m der Größenordnung von 8,5 mm liegt.

17. Intraokularlinse nach einem der vorhergehenden Anspruche, **dadurch gekennzeichnet, dass** der Durchmesser der optischen Abschnitt m der Großenordnung von 6,5 mm liegt.

18. Familie von Intraokularlinsen nach einem der Anspruche 1 bis 17, **dadurch gekennzeichnet, dass** jede Linse der Familie fur alle Leistungen zwischen -5 und +15 Dioptrien im Wesentlichen den gleichen axialen Abstand d besitzt

19. Familie von Intraokularlinsen nach einem der Anspruche 1 bis 17, **dadurch gekennzeichnet, dass** jede Linse der Familie fur alle Leistungen zwischen 0 und +10 Dioptrien im Wesentlichen den gleichen axialen Abstand d besitzt.

20. Familie von Intraokularlinsen nach Anspruch 19, **dadurch gekennzeichnet, dass** jede Linse der Familie im Wesentlichen den gleichen axialen Abstand d besitzt, der fur alle Leistungen zwischen -10 und -1 Dioptrien entsprechend zwischen etwa 1,74 mm und 1,24 mm veranderlich ist

## Claims

1. Intraocular lens for implantation in the capsular bag having a central optical part (10, 110) with a convex posterior face (11, 111) and a concave or planar anterior face and a peripheral haptic part (20), **characterised in that** the optical part is produced from flexible material and the axial distance d between the centre of the posterior face of the optical part and the anterior surface at the periphery of the optical part is greater than 0.8 mm.

2. Intraocular lens according to Claim 1, **characterised in that** the dioptre value of the optical part is between -5 and +15.

3. Intraocular lens according to Claim 1, **characterised in that** the dioptre value of the optical part is between -10 and +10.

4. Intraocular lens according to any one of the preceding claims, **characterised in that** the axial distance is greater than approximately 1.20.

5. Intraocular lens according to any one of Claims 1 to 4, **characterised in that** the axial distance is between approximately 1.20 and 1.28 mm.

6. Intraocular lens according to any one of Claims 1 to 4, **characterised in that** the axial distance is equal to or greater than 1.24 mm.

7. Intraocular lens according to any one of Claims 1 to 4, **characterised in that** the axial distance is between approximately 1.24 and 1.74 mm.

8. Intraocular lens according to any one of the preceding claims, **characterised in that** the optical part is produced entirely from flexible material.

9. Intraocular lens according to any one of the preceding claims, **characterised in that** the diameter of the optical part is between 6 and 7 mm approximately and the diameter defined by the periphery of the haptic part is 10.5 ± 0.5 mm.

10. Intraocular lens according to any one of the preceding claims, **characterised in that** the haptic part (20, 120) comprises three closed-contour loops, each comprising two arms (22, 122) extending roughly radially and a circumferential band (25, 125), whose angular extent is equal to or greater than 60°.

11. Intraocular lens according to any one of Claims 1 to 9, **characterised in that** the haptic part (120) comprises three closed-contour loops, each comprising two arms (122) extending roughly radially and a circumferential band (125) whose angular extent is around 80°.

12. Intraocular lens according to Claim 10 or 11, **characterised in that** the circumferential bands of the loops constitute segments (129) of a peripheral ring of other segment connecting the three circumferential bands to each other.

13. Intraocular lens according to Claims 10, 11 or 12, **characterised in that** the angular extent of the haptic part (20, 120) at the periphery of the optical part is equal to or even greater than 60°.

14. Intraocular lens according to any one of Claims 10 to 13, **characterised in that** the three loops are identical and have an angle of 120° with respect to each other.

15. Intraocular lens according to any one of the preceding claims, **characterised in that** the radius of curvature of the posterior face is greater than approximately 6 mm.

16. Intraocular lens according to Claim 15, **characterised in that** the radius of curvature of the posterior face is around 8.5 mm.

17. Intraocular lens according to any one of the preceding claims, **characterised in that** the diameter of the optical part is around 6.5 mm.

18. A family of intraocular lenses according to any one of Claims 1 to 17, **characterised in that** each lens in the family has substantially the same axial distance d for all the powers between -5 and +15 dioptres.

19. A family of intraocular lenses according to any one of Claims 1 to 17, **characterised in that** each lens in the family has substantially the same axial distance d for all the powers between 0 and +10 dioptres.

20. A family of intraocular lenses according to Claim 19, **characterised in that** each lens in the family has substantially the same axial distance d variable for all the powers between -10 and -1 dioptre, respectively, between approximately 1.74 mm and 1.24 mm.
